# EUROPEAN PATENT SPECIFICATION

(11) **EP 0 463 484 B1**
(45) Date of publication and mention of the grant of the patent: **12.03.1997**
(21) Application number: 91109672.5
(22) Date of filing: 04.07.1991
(51) Int. Cl.: C07C 273/16

(54) **Vacuum separators for use in urea plants**
Vakuumabscheidern für Harnstoff-Fabrikationsanlagen
Séparateurs sous vide pour usines à production d'urée

(43) Date of publication of application: 02.01.1992
(73) Proprietor: UREA CASALE S.A., CH-6900 Lugano-Besso (CH)
(72) Inventor: Pagani, Giorgio, CH-6900 Lugano (CH)
(74) Representative: Checcacci, Giorgio

(56) References cited:
- EP-A- 0 302 213
- CH-A- 340 503

## Description

The present invention refers to vacuum separators for use in avoiding the draggings of urea and eliminating the formation of fouling substances in the vacuum separation from the urea solution, of the urea, by distillation of this last solution in two stages for getting fused urea with negligible residues of water and for vacuum separation of distilled vapors in two separation stages at different temperatures and pressures, in which the solution of fused urea is recovered in the lower zone, while the distilled vapors are collected in the superior zone of said separators.

As it is known, the urea formation water obtained by dehydration of ammonium carbamate in the synthesis reactor is removed in the vacuum distillation section, in which the urea solution is usually distilled in two stages: the first stage at the pressure of about 0.3ö0.4 bar a, and the following stage at the pressure of 0.05 bar a, obtaining in this manner fused urea with 99.7% by weight of urea and 0.3% by weight of water.

The distilled vapors are separated on its turn from the urea solution usually in two vacuum separation stages; the first separation stage of the urea-solution holding 95ö96% by weight of urea and the second separation stage from fused urea with 99.7% by weight of urea, and they are sent to the condensation according to known technics.

On its turn the fused urea coming out from the distillation section is conveyed to the crystallization phase (prilling or granulation) for getting the finished product.

The distilled vapors are substantially constitued by overheated water steam holding residues of NH₃ and CO₂. The lowering of the water steam tension in the urea solution causes the generated water steam to be in thermal balance with the urea solution, but really it is overheated with respect to the process pressure.

The operative conditions in the inside of the two vacuum separators normally used and widely described in the Prior Art are the following ones :
- First separator :: Pressure: P = 0.3ö0.4 bar Temperature: T = 130°C Saturation temperature: T = 69ö75°C
- Second separator :: Pressure: P = 0.05 bar Temperature: T = 140°C Saturation temperature: T = 32°C

Disadvantageously the separation between liquid and vapor obtained in separators of cyclone type, that are normally used in these separations, is not optimal, as the vapors hold still some microscopic drops of urea that are dragged by the same vapors in the condensation section, causing so problems of product loss and pollution in the case of unloading of final condensates in sewers.

Therefore these condensates (unloading water) must be treated in complex and costly treatment plants, in which the urea contained therein submitted to hydrolysis and NH₃ and CO₂ formed in this manner are separated by distillation together with the other quantities of the same substances hold in condensates.

Further these drops, coming in contact with colder zones of vapors, e.g. separator walls, output pipe, tend to deposit, solidifying or causing foulings.

In particular the phenomena of urea dragging in distilled vapors are usually predominant in the first separator, as the urea solution is rich of water, while the second vacuum separation stage, where the temperatures are higher (superior to the temperature of urea fusion), holds a water quantity that is practically zero in fused urea if compared with the first stage of vacuum separation and it shows the major problems of foulings as the urea dragged from vapors tends to generate condensation products insoluble in water, e.g. urates, cyanates and isocyanates, whose fouling increases in time, causing so obstructions compromising the vapor passage and causing the necessity of frequent and costly maintenance works.

One of the actions normally made for limiting the negative effects of the phenomenon of foulings is the reduction of the temperature difference between vapors and walls to a minimum value, accurately insulating all of the walls in contact with vapors.

Nevertheless, also acting in this manner, the phenomenon of foulings is slowed down in time, but it is not avoided completely and costly maintenance interventions are always required.

Alternatively, it has been proposed by EP-A-0 302 213 to bring the vapors containing NH₃ and CO₂ and urea entrainments coming from the separators, into intimate contact with an aqueous urea solution acting as a washing solution.

Therefore it is important to solve in a final manner the problem of urea dragging and of the related foulings.

The technical problem underlying the present invention is to provide a vacuum separator in which the above mentioned drawbacks are avoided and the fused urea is obtained with minimum water residues.

This problem is solved, according to the invention, by a vacuum separator as defined in appended claims 1 and 2.

According to a first embodiment of the invention a cyclone-type vacuum separator, comprising an annular wall coaxially located within an internal wall thereof and forming an annulus therewith, is characterized in that it further comprises:
a) a first annular element coaxially located within said annular wall, said first annular element having a lower end welded to said annulus-forming wall, thereby defining a first jacket having the form of an open pocket closed on bottom, the upper end of said first element being free and having a diameter smaller than that of the lower end thereof;
b) a second element, having the form of a dome overhanging the first element, said second element having a lower end located at a height lower than that of the upper end of said first element and having a diameter greater than that of said first element, a second jacket being provided between said first and second elements;
c) third elements for the distribution of a washing liquid, located within said second jacket;
d) pipe means for withdrawing an urea-enriched washing liquid from the separator, located on the outer periphery of said first jacket.

According to a second embodiment of the invention, a gravity-type vacuum separator, comprising a vapour deflecting element located within an internal wall thereof above the heading of a vacuum distiller, comprises a first annular element having a lower end which is directly connected to the internal wall thereof.

A particularly simple and advantageous embodiment foresees that said first and second elements are formed by a series of walls welded with one another and said third elements are sprayers or nebulizers. The second element is further provided in its inside with a diffuser cone for a gradual and optimal conveying of distilled vapors coming from the annulus of separator.

Said first element holds also resistances for the thermal insulation between the colder liquid, that is located in the inside of said first jacket, and the hotter vapors holding urea passing in the inside of the separator, for avoiding the cooling of this last one and then avoiding foulings, also if it can be foreseen that the eventual urea foulings, formed on the walls of said first element, act as a real further thermal insulation material.

Working in this manner numerous advantages are obtained and among them we mention in particular the following ones :
- The nearly total elimination (99%) of urea dragged in vapors;
- A reduction of the temperature of vapors with following lock of degradation phenomena, e.g. formation of urates, residues of urea still present;
- An enrichment of vapors coming out of the washing zone with water;
- The reduction of vortexes formation, that facilitates the deposit of solid urea with danger of degradation in the "dead" zones that are not touched by vapors.

These and other advantages prevent the pollution of condensates, avoiding in the same time the product losses, and further the deposit of solid urea on the walls of the separator and allow to make a further vapor/liquid/solid separation in a demister of the conventional type, e.g. of lamina type, without causing obstruction problems.

Advantageously a part of the washing water (or a part of an acqueous solution of urea) shall evaporate eliminating the overheating of vapor, while the remaining part, holding the passivated urea during the removal operation, shall be recycled to other plant sections by means of a proper pipe.

The above mentioned drawbacks are avoided owing to the creation of optimal operative conditions for getting the removal of urea draggings and the absence of foulings, avoiding the use of complex and expensive plants for the treatment of condensates and keeping the temperature and concentration of the fused urea solution within the wished limits without causing unpleasant variations of the product quantity and of the urea production.

The different characteristics and advantages of the invention shall appear better in the following detailed description of the embodiments represented in the annexed drawings, in which:
- Figure 1 shows a partial and schematic section view in axial planes of a separator belonging to a vacuum distillation stage, that is present in the mayor number of existing urea plants of traditional type.
- Figure 2 is a view similar to the Figure 1 of an embodiment of cyclone type separator of the invention with the indication of new devices, suitable for avoiding the urea draggings and avoiding each possible fouling of the walls of the same separators.
- Figure 3 is a section view similar to Figure 1, but related to a conventional gravity separator.
- Figure 4 is a view of the embodiment arising from the application of the invention on the gravity separator of the type shown in Figure 3.

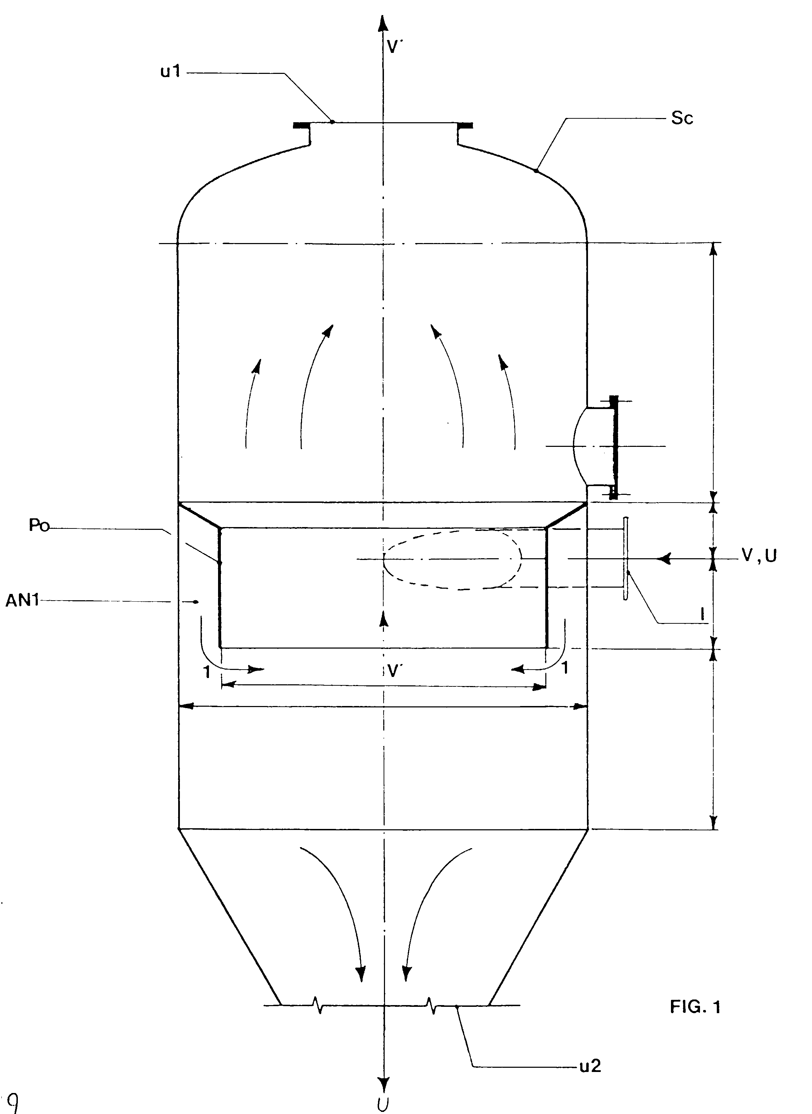

In particular the Figure 1 represents a traditional separator (Sc) of the cyclone type, provided with annulus (AN1) for the input of vapors (V) and of the fused urea solution (U) coming from a distiller external to the separator and not represented.

The vapors (V) entering the separator (Sc) through the input (I) go down near the annulus (AN1) along the path 1, and then go up again in the central part of the separator (Sc) dragging therewith the drops of fused urea and going out from (Sc) through the opening (u1) under the form of polluted vapors V'.

When the fused urea (U) is gone down along the path 1, it goes out of the separator (Sc) through the opening (u2).

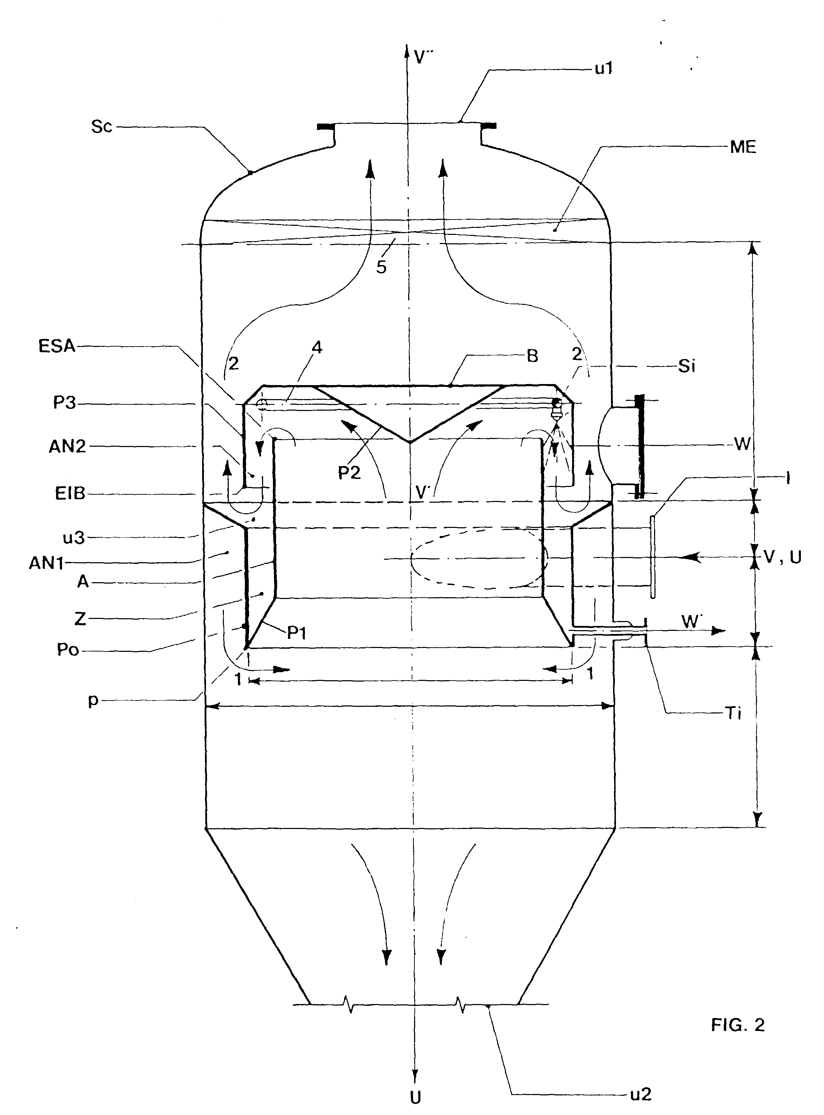

The Figure 2 shows a separator (Sc) of the cyclone type, as described in Figure 1, that now embodies, according to the invention, in addition to a demister (ME) of conventional type, a first (A) and a second (B) element respectively, sprayers (Si) for the distribution of the washing liquid (W) and pipes (Ti) for the unloading of the washing liquid enriched with urea (W').

The element (A) forms a first open pocket jacket (Z), closed in its bottom with the wall (Po) forming the annulus (AN1), to which it is welded in (p). The element (B) is located on its turn over the element (A) for forming a washing annulus (AN2) between the lower end (EIB) of (B) and the superior end (ESA) of (A).

The distilled vapors dragging the urea (V'), after having crossed the annulus (AN1) along the path 1, are dragged upwards centrally into the separator by means of proper walls (P1), (P2) and (P3) and piped towards the bottom in the washing annulus (AN2), where they are collided by (W) for removing the urea residues.

The vapors washed in this manner, enriched with water and partially cooled (V''), being in conditions that cannot cause foulings, pass across the opening (u3) and go up along the path 2 and then go out from (Sc), after having crossed the demister (ME) through the opening (u1).

The washing liquid enriched with urea (W') is collected in the jacket (Z) and leaves the separator (Sc) through the pipes (Ti).

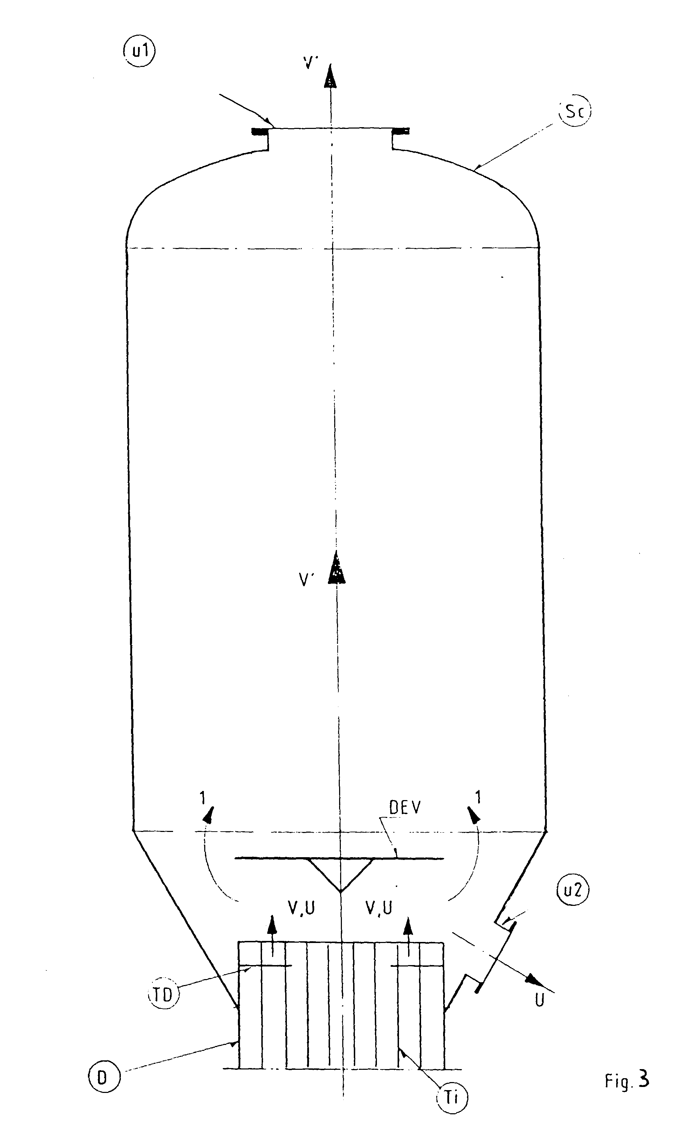

The Figure 3 represents a traditional separator (Sc) of the gravity type, that on the contrary embodies an element (DEV) for the deviation of vapors (V) and the heading (TD) of the vacuum distiller (D) having a series of pipes (Ti).

The vapors (V) going out from (TD) are collected directly in the separator (Sc), cross the element (DEV) and go up along the path 1, dragging therewith the drops of fused urea towards the opening (u1) under the form of polluted vapors V'.

The fused urea (U) coming from the distiller (D) similarly to (V), on its turn goes out from the separator (SC) through the opening (u2) under the gravity action.

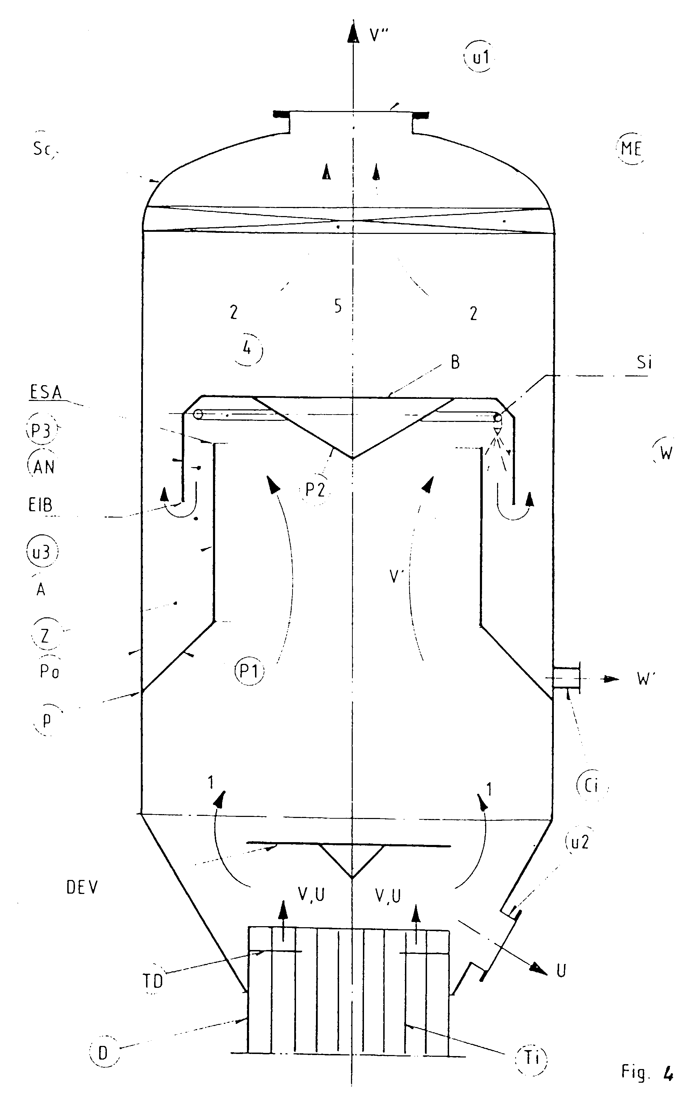

The Figure 4 represents a separator (Sc) of the gravity type, as described in Figure 3, embodying now, according to the invention, besides the element (DEV), a demister (ME) of conventional type, a first (A) and a second element (B) respectively, sprayers (Si) for the distribution of washing liquid (W) and pipes (Ci) for the unloading of the washing liquid enriched with urea (W').

Also in this case the element (A) forms a first open pocket jacket (Z), closed in its bottom, together with the internal wall (Po) of the vacuum separator (Sc) to which it is welded in (p).

The element (B) is located on its turn over the element (A) for forming a washing annulus (AN) between the lower end (EIB) of (B) and the superior end (ESA) of (A).

The distilled vapors dragging the urea (V'), after having crossed the element (DEV) along the path 1, guided by the proper walls (P1), (P2) and (P3), are dragged centrally into the separator and piped towards the bottom in the washing annulus (AN) where they are collided by (W) for removing the urea residues. The vapors washed in this manner, enriched with water and partially cooled (v'') pass, being in conditions that cannot cause the foulings, across the opening (u3) and go up along the path 2 and then go out from (Sc), after having crossed the demister (ME) through the opening (u1).

The washing liquid enriched with urea (W') is collected in the jacket (Z) and leaves the separator (Sc) through the pipes (Ci).

## Claims

1. A cyclone-type vacuum separator (Sc) of vapours generated by the distillation of an urea solution, comprising an annular wall (Po) coaxially located within an internal wall thereof and forming an annulus (AN1) therewith, characterized in that it further comprises:
a) a first annular element (A) coaxially located within said annular wall (Po), said first annular element (A) having a lower end (p) welded to said annulus-forming wall (Po), thereby defining a first jacket (Z) having the form of an open pocket closed on bottom, the upper end (ESA) of said first element (A) being free and having a diameter smaller than that of the lower end (p) thereof;
b) a second element (B), having the form of a dome overhanging the first element (A), said second element (B) having a lower end (EIB) located at a height lower than that of the upper end (ESA) of said first element (A) and having a diameter greater than that of said first element (A), a second jacket (AN2) being provided between said first (A) and second (B) elements;
c) third elements (Si) for the distribution of a washing liquid (W), located within said second jacket (AN2);
d) pipe means (Ti) for withdrawing an urea-enriched washing liquid (W') from the separator (Sc), located on the outer periphery of said first jacket (Z).

2. A gravity-type vacuum separator (Sc) of vapours generated by the distillation of an urea solution, comprising a vapour deflecting element (DEV) located within an internal wall (Po) thereof above the heading (TD) of a vacuum distiller (D), characterized in that it further comprises:
a) a first annular element (A) coaxially located within said internal wall (Po), said first annular element (A) having a lower end (p) connected to said wall (Po), thereby defining a first jacket (Z) having a form of an open pocket closed on bottom, the upper end (ESA) of said first element (A) being free and having a diameter smaller than that of the lower end (p) thereof;
b) a second element (B), having the form of a dome overhanging the first element (A), said second element (B) having a lower end (EIB) located at a height lower than that of the upper end (ESA) of said first element (A) and having a diameter greater than that of said first element (A), a second jacket (AN) being provided between said first (A) and second (B) elements;
c) third elements (Si) for the distribution of a washing liquid (W), located within said second jacket (AN);
d) pipe means (Ci) for withdrawing an urea-enriched washing liquid (W') from the separator (Sc), located on the outer periphery of said first jacket (Z).

3. A vacuum separator according to anyone of claims 1 or 2, characterized in that said first (A) and second (B) elements comprise a plurality of walls welded to one another.

4. A vacuum separator according to anyone of claims 1 or 2, characterized in that said third elements (Si) are sprayers or nebulizers.

5. A vacuum separator according to anyone of claims 1 or 2, characterized in that said second element (B) is provided in its inside with a diffuser cone (P2) for a gradual and optimal conveying of vapours.

6. A vacuum separator according to anyone of claims 1 or 2, characterized in that it further comprises a laminar demister (ME).

7. A vacuum separator according to anyone of claims 1 or 2, characterized in that said first element (A) is provided in its inside with a thermal insulating material.

8. A vacuum separator according to claim 7, characterized in that said thermal insulating material comprises urea foulings formed on the walls of said first element (A).

## Patentansprüche

1. Vakuumabscheider der Zyklon-Bauart zum Abscheiden von Dämpfen, die bei der Destillation einer Harnstofflösung erzeugt werden, mit einer Ringwand (Po), die koaxial innerhalb einer Innenwand des Vakuumabscheiders gelegen ist und damit einen Ringraum (AN1) bildet, gekennzeichnet durch
a) ein erstes ringförmiges Element (A), das koaxial mit der Ringwand (Po) ist und ein unteres Ende (p) hat, das mit der den Ringraum bildenden Ringwand (Po) verschweißt ist, um so eine erste Umhüllung (Z) in Form einer nach oben offenen, am Boden geschlossenen Tasche zu bilden, wobei das obere Ende (ESA) des ersten Ringelementes (A) freiliegt und einen kleineren Durchmesser als dessen unteres Ende (p) hat;
b) ein zweites Element (B) in Gestalt eines das erste Element (A) überhängenden Domes, der ein unteres Ende (EIB) hat, das auf einem Niveau unterhalb des oberen Endes (ESA) des ersten Elementes (A) liegt, und einen größeren Durchmesser als das erste Element (A) aufweist, wobei eine zweite Umhüllung (AN2) zwischen dem ersten Element (A) und dem zweiten Element (B) gebildet ist;
c) dritte Elemente (Si) zum Verteilen einer Waschflüssigkeit (W), die innerhalb der zweiten Umhüllung (AN2) gelegen sind;
d) Rohrleitungsmittel (Ti) zum Abziehen einer mit Harnstoff angereicherten Waschflüssigkeit (W') aus dem Abscheider (Sc), die am äußeren Umfang der ersten Umhüllung (Z) angeordnet sind.

2. Vakuumabscheider (Sc) der Schwerkraft-Bauart zum Abscheiden von Dämpfen, die durch Destillation einer Harnstoff-Lösung erzeugt werden, umfassend ein Dampf ableitendes Element (DEV), das innerhalb einer Innenwand (Po) oberhalb des Kopfes (TD) eines Vakuum-Destillierers (D) angeordnet ist, gekennzeichnet durch:
a) ein erstes ringförmiges Element (A), das koaxial innerhalb der besagten Innenwand (Po) angeordnet ist und ein mit dieser verbundenes unteres Ende (p) aufweist, wodurch eine erste Umhüllung (Z) in Gestalt einer offenen, am Boden geschlossenen Tasche gebildet wird, wobei das obere Ende (ESA) des ersten Ringelementes (A) freiliegt und einen kleineren Durchmesser als dessen unteres Ende (p) aufweist;
b) ein zweites Element (B) in Gestalt eines das erste Element (A) überhängenden Domes, wobei das zweite Element (B) ein unteres Ende (EIB) hat, das auf einem niedrigeren Niveau als das obere Ende (ESA) des ersten Elementes (A) liegt und einen größeren Durchmesser als das erste Element (A) hat, wobei eine zweite Umhüllung (AN) zwischen dem ersten Element (A) und dem zweiten Element (B) gebildet ist;
c) dritte Elemente (Si) zur Verteilung einer Waschflüssigkeit (W), die innerhalb der besagten zweiten Umhüllung (AN) angeordnet sind;
d) Rohrleitungsmittel (Ci) zum Abziehen einer mit Harnstoff angereicherten Waschflüssigkeit (W') aus dem Abscheider (Sc), die am äußeren Umfang der ersten Umhüllung (Z) angeordnet sind.

3. Vakuumabscheider nach Anspruch 1 oder 2, dadurch gekennzeichnet, daß die ersten und zweiten Elemente (A,B) mehrere miteinander verschweißte Wände aufweisen.

4. Vakuumabscheider nach Anspruch 1 oder 2, dadurch gekennzeichnet, daß die dritten Elemente (Si) Sprüh- oder Zerstäubungsvorrichtungen sind.

5. Vakuumabscheider nach Anspruch 1 oder 2, dadurch gekennzeichnet, daß das besagte zweite Element (B) auf seiner Innenseite mit einem Diffusorkonus (P2) zum graduellen und optimalen Fördern von Dämpfen versehen ist.

6. Vakuumabscheider nach Anspruch 1 oder 2, dadurch gekennzeichnet, daß er ferner einen laminaren Tropfenabscheider (ME) aufweist.

7. Vakuumabscheider nach Anspruch 1 oder 2, dadurch gekennzeichnet, daß das erste Element (A) auf seiner Innenseite mit einem thermisch isolierendem Material beschichtet ist.

8. Vakuumabscheider nach Anspruch 7, dadurch gekennzeichnet, daß das thermisch isolierende Material Harnstoff-Anwachsungen umfaßt, die auf den Wänden des ersten Elementes (A) geformt sind.

## Revendications

1. Séparateur sous vide du type séparateur à cyclone (Sc), de vapeurs produites par la distillation d'une solution d'urée, comprenant une paroi annulaire (Po) située de façon coaxiale à l'intérieur d'une paroi interne de celui-ci et formant un anneau (AN1) avec celle-ci, caractérisé en ce qu'il comprend en outre :
a) un premier élément annulaire (A) situé de façon coaxiale à l'intérieur de ladite paroi annulaire (Po), ledit premier élément annulaire (A) ayant une extrémité inférieure (p) soudée à ladite paroi formant anneau (Po), définissant ainsi une première chemise (Z) ayant la forme d'une poche ouverte fermée au fond, l'extrémité supérieure (ESA) dudit premier élément (A) étant libre et ayant un diamêtre plus petit que celui de son extrémité inférieure (p);
b) un second élément (B), ayant la forme d'un dôme surplombant le premier élément (A), ledit second élément (B) ayant une extrémité inférieure (EIB) située à une hauteur inférieure à celle de l'extrémité supérieure (ESA) dudit premier élément (A) et ayant un diamêtre plus grand que celui dudit premier élément (A), une seconde chemise (AN2) étant pourvue entre ledit premier élément (A) et ledit second élément (B) ;
c) des troisièmes éléments (Si) pour la distribution d'un liquide de lavage (W), situés à l'intérieur de ladite seconde chemise (AN2) ;
d) des tubes (Ti) pour retirer un liquide de lavage (W') enrichi en urée, du séparateur (Sc), situés sur la périphérie extérieure de ladite première chemise (Z).

2. Séparateur sous vide du type séparateur par gravité (Sc), de vapeurs produites par la distillation d'une solution d'urée, comprenant un élément déflecteur de la vapeur (DEV) situé à l'intérieur d'une paroi interne (Po) du séparateur, au-dessus de la tête d'un appareil de distillation sous vide (D), caractérisé en ce qu'il comprend en outre :
a) un premier élément annulaire (A) situé de façon coaxiale à l'intérieur de ladite paroi annulaire (Po), ledit premier élément annulaire (A) ayant une extrémité inférieure (p) reliée à ladite paroi (Po), définissant ainsi une première chemise (Z) ayant la forme d'une poche ouverte fermée au fond, l'extrémité supérieure (ESA) dudit premier élément (A) étant libre et ayant un diamêtre plus petit que celui de son extrémité inférieure (p) ;
b) un second élément (B), ayant la forme d'un dôme surplombant le premier élément (A), ledit second élément (B) ayant une extrémité inférieure (EIB) située à une hauteur inférieure à celle de l'extrémité supérieure (ESA) dudit premier élément (A) et ayant un diamêtre plus grand que celui dudit premier élément (A), une seconde chemise (AN) étant pourvue entre ledit premier élément (A) et ledit second élément (B) ;
c) des troisièmes éléments (Si) pour la distribution d'un liquide de lavage (W), situés à l'intérieur de ladite seconde chemise (AN) ;
d) des tubes (Ci) pour retirer un liquide de lavage (W') enrichi en urée, du séparateur (Sc), situés sur la périphérie extérieure de ladite première chemise (Z).

3. Séparateur sous vide selon l'une quelconque des revendications 1 ou 2, caractérisé en ce que ledit premier élément (A) et ledit second élément (B) comprennent une pluralité de parois soudées les unes aux autres.

4. Séparateur sous vide selon l'une quelconque des revendications 1 ou 2, caractérisé en ce que lesdits troisièmes éléments (Si) sont des vaporisateurs ou des nébuliseurs.

5. Séparateur sous vide selon l'une quelconque des revendications 1 ou 2, caractérisé en ce que ledit second élément (B) est pourvu intérieurement d'un cône de diffusion (P2) pour un acheminement graduel et optimal des vapeurs.

6. Séparateur sous vide selon l'une quelconque des revendications 1 ou 2, caractérisé en ce qu'il comprend en outre un dispositif anti-buée laminaire (ME).

7. Séparateur sous vide selon l'une quelconque des revendications 1 ou 2, caractérisé en ce que ledit premier élément (A) est pourvu intérieurement d'un matériau d'isolation thermique.

8. Séparateur sous vide selon la revendication 7, caractérisé en ce que ledit matériau d'isolation thermique comprend des incrustations d'urée formées sur les parois dudit premier élément (A).
